# EUROPEAN PATENT SPECIFICATION

(11) **EP 2 389 210 B1**
(45) Date of publication and mention of the grant of the patent: **12.06.2013**
(21) Application number: 10702453.1
(22) Date of filing: 15.01.2010
(51) Int. Cl.: A61M 5/28, A61M 5/24

(54) **MEDICAMENT IDENTIFICATION SYSTEM FOR MULTI-DOSE INJECTION DEVICES**
MEDIKAMENTENIDENTIFIKATIONSSYSTEM FÜR MEHRFACHDOSIERUNGSINJEKTIONSVORRICHTUNGEN
Système d'identification de médicaments pour dispositifs d'injection de doses multiples

(30) Priority: 23.01.2009 US 146768 P; 20.02.2009 EP 09002398
(43) Date of publication of application: 30.11.2011
(73) Proprietor: Sanofi-Aventis Deutschland GmbH, 65929 Frankfurt (DE)
(72) Inventor: JANSEN, Paul Edward, 65926 Frankfurt am Main (DE); GURAL, Richard, Bridgewater, New Jersey 08807 (US)
(86) International application number: PCT/EP2010/050443
(87) International publication number: WO 2010/084084

(56) References cited:
- EP-A- 1 946 787
- WO-A-02/11664
- WO-A-03/047657
- WO-A-03/086511
- WO-A-2005/077673
- US-A- 6 120 481
- US-B1- 6 338 200

## Description

### Field of the Invention

The present invention relates to multi-dose injection devices, more specifically to injectors of the kind that provide for administration of medicinal products from a multi-dose cartridge, sometimes referred to as pen-type injectors. In particular, the present invention relates to an identification system for such injectors where the user can easily determine or distinguish the type of medication contained within the cartridge by visual observation or tactile feel.

### Background of the Invention

Pen-type injectors are well known and all universally use some form of cartridge capable of delivering multiple doses of a specific type of medicine, such as human growth hormone or insulin. For a number of end users of such devices (typically patients being prescribed medicines) several injectors are needed to dispense a number of different medicaments. For example, diabetic patients may need one injection device containing long lasting insulin and a second injector containing short acting insulin. Clearly, it is important for such patients to know with absolute certainty what medicine is contained within which injection device. This especially true for elderly patients and those that are visually impaired. Although manufacturers of medicament cartridges typically use some form of identification (lettering, color and/or symbols) on the labels affixed to the cartridges, this form of identification is often subtle and not readily apparent to certain types of users of such devices. One manufacturer has previously used small plastic chips of various colors so a user can connect to a portion of the device as an identification of the medicament. Unfortunately, these chips are very small and not easily replaceable. Another manufacturer, as explained in U.S. Patent No. 5,693,027, supplies a color-coded adaptor top to fit on the end of the cartridge to assist in distinguishing the medicament. Again, these adaptor tops are relatively small and not distinct enough to allow certain users to easily recognize the medicament contained in the device. Some manufacturers of certain types of disposable injectors color various parts of the device, like the housing and cap, in an attempt to distinguish devices containing different medicaments, but again the variations in color are often subtle and not easily recognized by particular users. Accordingly, there exists a strong need to provide users of such devices simple and clear means to determine and distinguish the type of medicine that is contained in the devices. Moreover, it is important to allow the user to select and apply the identification so they have a stronger association with that form of identification as it relates to a specific medicament.

The invention solves the above-described problems by providing two types of medicament identification means, one being an elastic band that a user places around the device and another that is a unique structural design of the pocket clip attached to the device. Both identification systems provide the user of the device with a clear and simple visual and/or tactile form of identifying the particular medicament contained in the device. These and other advantages will become evident from the following more detailed description of the invention.

### Summary of the Invention

According to a first aspect of the invention there is provided a medication identification system for a multi-dose injection device that comprises a pen-type injector having both distal and proximal ends. A cartridge holder is located at the distal end that is configured to accept a cartridge of medicament. An elastic identification band, removably positioned and frictionally held on the cartridge holder or on the proximal end of the device, has an outer surface that serves as an identifier of the medicament contained in the cartridge. The band can be made of any type of material provided that it is elastic, removable and capable of being stretched to fit around the outside of the injection device, much in the same way a conventional rubber band holds together a roll of paper. Preferably, the band is fabricated from a polymeric material and in addition to the identification aspect, functions to provide the user with a gripping surface to aid in the injection process. Such a preferred band is similar to what is used on a variety ballpoint pens, with the exception that the invention is configured to be easily removed or exchanged with a different band by the end user of the device.

In one embodiment of the identification system, the outer surface of the band can be pigmented with a color to allow a user of the injection device to visually identify and distinguish the device as containing a specific type of medicament. Several colored bands can be provided to allow a user to associate a particular color with a specific medicament. For example, a green colored band could designate short acting insulin and a yellow band would designate long acting insulin. Once a new cartridge of medicament is inserted into the injection device the user would then place the correct colored band on the device corresponding to that particular medicament. By allowing the user to select and add the identification means to the device this greatly increases the chance that the user will remember the association between the identifier and the specific medicament.

In those circumstances where the user's eyesight is impaired, then the outer surface of the band can be textured to tactilely identify and distinguish the device as containing a specific type of medicament. This texturing can take any form, such as a raised design or even lettering, like Braille, provided that the user can easily recognize it. Both coloring and texturing could be also be used on a single band. In some cases the use of multiple bands might be beneficial to a particular patient.

Another embodiment of the invention for a medication identification system for a multi-dose injection device comprises using a pocket clip attached to the proximal end of the injection device or to a removable cap covering the cartridge holder, where the pocket clip has a very unique and easily identifiable structural design that functions as an identifier of the medicament contained in the cartridge. The structural design of the pocket clip is configured to allow the user to tactilely identify and distinguish the device as containing a specific type of medicament. The clip can be fabricated of metal or a synthetic material, such as a polymeric plastic, and can be molded or bent to create a unique and distinguishing structural design that is both visually and tactilely apparent to the user, and that allows the user to associate the design with a specific medicament. The pocket clip can also be interchangeable with other clips having different structural designs to allow the user a choice of several designs to choose from, thus increasing the chance that the user will remember the association of the chosen design with a specific medicament.

These and other aspects of the invention will become more apparent from the detail description of the preferred embodiment contained below and will be described with reference to the accompanying drawings.

### Brief Description of the Drawings

FIG. 1 shows a perspective view of an identification band in accordance with the present invention;
FIG. 2 shows a side view of a pen-type injector having attached thereto an identification band in accordance with the present invention; and
FIG. 3 shows a side view of a pen-type injector having attached thereto a pocket clip identification system in accordance with the present invention;

### Detailed Description of Preferred Embodiments

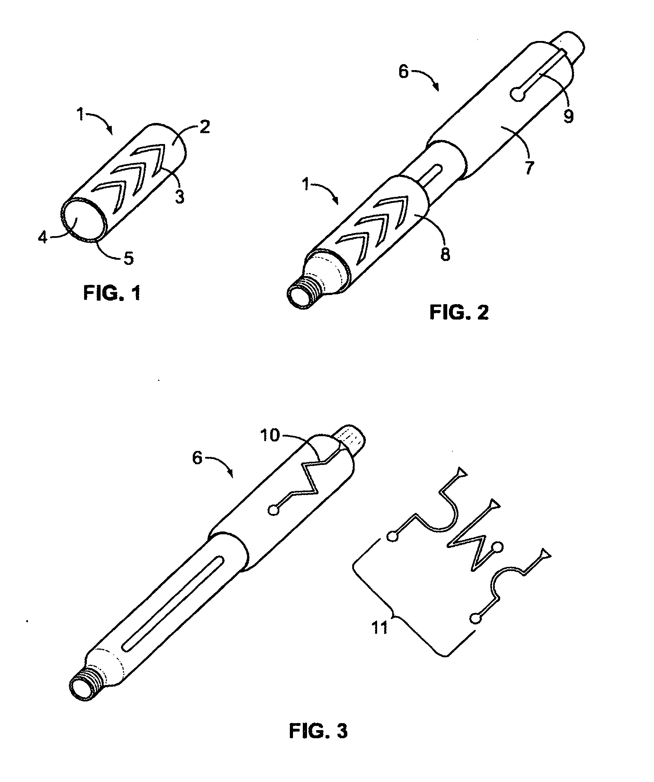

Referring first to FIG. 1, there is shown the elastic band 1 of one embodiment of the medicament identification system having an outer surface 2 and an annular inner surface 4 separated from the outer surface by a wall 5. The material used to construct the band is preferably a polymeric material that exhibits the flexible and elastic memory properties, similar to that of a conventional rubber band. This will allow a user to stretch or enlarge inner surface 4 so the band can be slipped over and onto the outer wall or housing of injection device 6, for example as illustrated in FIG. 2. The length of the band is variable and can be selected depending on the design of injection device 6 and the position/location where it is to be placed on the device.

The band can be pigmented with a color or colors that is associated with a particular medicament and that the user can easily visualize. The particular method or color used to pigment the band is not critical to the invention; however, bright colors are particularly preferred for users with poor or limited vision. The inner surface may be textured or contain a chemical agent to assist in the band's ability to grip the injection device. The band may also contain texturing 3 on outer surface 2 to provide a gripping surface for the user. This texturing can be any design or writing, such as Braille, and is preferably a unique shape to further allow the user to identify the medicament in the injection device. This becomes important when the user is colorblind or has little to no eyesight. Although band 1 is shown in FIG. 2 as being located on the distal end of device 6, specifically on cartridge holder 8, it could also be placed on the proximal end of the device near pocket clip 9. Likewise, the band could be placed on a removable cap.

FIG. 3 illustrates a second embodiment of the invention where the identification system comprises an injection device 6 fitted with pocket clip 10 that has a unique structural design that allows a user to associate the design with a particular medicament. Alternate shapes of the pocket clip, such as those shown collectively as item 11 in FIG. 3, can be used for different medicaments. The specific design is not critical to the invention provided it allows a user who is visually impaired to tactically recognize the design. The clip may be removable and replaceable with different designed clips, each associated with a particular medicament.

The foregoing description of the specific embodiments will so fully reveal the general nature of the invention that others can, by applying current knowledge, readily modify and/or adapt for various application such specific embodiments without departing from the generic concept, and therefore such adaptations and modifications are intended to be comprehended within the meaning and range of equivalents of the disclosed embodiments. It is to be understood that the phraseology or terminology herein is for the purpose of description and not of limitation.

The means, materials, and steps for carrying out various disclosed functions may take a variety of alternative forms without departing from the invention. Thus, the expressions "means to ... " and "means for ... ", or any method step language as may be found in the specification above or the claims below, followed by a functional statement, are intended to define and cover whatever structural, physical, chemical or electrical element or structure, or whatever method step, which may now or in the future exist which carries out the recited function, whether or not precisely equivalent to the embodiment or embodiments disclosed in the specification above, i.e., other means or steps for carrying out the same function can be used; and it is intended that such expressions be given their broadest interpretation within the terms of the following claims.

## Claims

1. A medication identification system for a multi-dose injection device comprising,
a. a pen-type injection device with distal and proximal ends;
b. a cartridge holder located at the distal end configured to accept a cartridge of medicament; and
c. an elastic identification band removably positioned and frictionally held on the cartridge holder or proximal end of the device and having an outer surface that is an identifier of the medicament contained in the cartridge.

2. The identification system of claim 1 wherein the outer surface of the band is pigmented to allow a user of the injection device to visually identify and distinguish the device as containing a specific type of medicament.

3. The identification system of claim 1 wherein the outer surface of the band is textured to allow a user of the injection device to tactilely identify and distinguish the device as containing a specific type of medicament.

4. A medication identification system for a multi-dose injection device comprising,
a. a pen-type injection device with distal and proximal ends;
b. a cartridge holder located at the distal end configured to accept a cartridge of medicament; and
c. a pocket clip attached to the proximal end of the device or to a removable cap covering the cartridge holder having a structural design that is an identifier of the medicament contained in the cartridge.

5. The identification system of claim 4 wherein the structural design of the pocket clip is configured to tactilely identify and distinguish the device as containing a specific type of medicament.

6. The identification system of claim 4 wherein the clip is removably attached to the injection device to allow a user to interchange different pocket clips having different structural designs.

## Patentansprüche

1. Medikamentenidentifikationssystem für eine Mehrfachdosierungsinjektionsvorrichtung mit
a. einer stiftartigen Injektionsvorrichtung mit einem distalen und einem proximalen Ende,
b. einem Kartuschenhalter, der am distalen Ende angeordnet und so konfiguriert ist, dass er eine Arzneimittelkartusche aufnimmt, und
c. einem elastischen Identifikationsband, das entfernbar am Kartuschenhalter oder am proximalen Ende der Vorrichtung positioniert und reibungsmäßig daran gehalten ist und eine Außenfläche aufweist, die ein Identifizierungsmerkmal des in der Kartusche enthaltenen Arzneimittels ist.

2. Identifikationssystem nach Anspruch 1, wobei die Außenfläche des Bands pigmentiert ist, damit ein Benutzer der Injektionsvorrichtung die Vorrichtung optisch identifizieren und unterscheiden kann, dass sie eine spezifische Art von Arzneimittel enthält.

3. Identifikationssystem nach Anspruch 1, wobei die Außenfläche des Bands strukturiert ist, damit ein Benutzer der Injektionsvorrichtung die Vorrichtung haptisch identifizieren und unterscheiden kann, dass sie eine spezifische Art von Arzneimittel enthält.

4. Medikamentenidentifikationssystem für eine Mehrfachdosierungsinjektionsvorrichtung mit
a. einer stiftartigen Injektionsvorrichtung mit einem distalen und einem proximalen Ende,
b. einem Kartuschenhalter, der am distalen Ende angeordnet und so konfiguriert ist, dass er eine Arzneimittelkartusche aufnimmt, und
c. einem Taschenclip, der am proximalen Ende der Vorrichtung oder an einer abnehmbaren, den Kartuschenhalter abdeckenden Kappe angebracht ist, mit einem Strukturdesign, das ein Identifizierungsmerkmal des in der Kartusche enthaltenen Arzneimittels ist.

5. Identifikationssystem nach Anspruch 4, wobei das Strukturdesign des Taschenclips so konfiguriert ist, damit haptisch identifiziert und unterschieden werden kann, dass die Vorrichtung eine spezifische Art von Arzneimittel enthält.

6. Identifikationssystem nach Anspruch 4, wobei der Clip abnehmbar an der Injektionsvorrichtung angebracht ist, damit ein Benutzer unterschiedliche Taschenclips mit unterschiedlichen Strukturdesigns austauschen kann.

## Revendications

1. Système d'identification de médicaments pour un dispositif d'injection de doses multiples, comprenant:
a. un dispositif d'injection de type crayon avec des extrémités distale et proximale;
b. un support de cartouche placé à l'extrémité distale, configuré pour accepter une cartouche de médicament; et
c. une bande d'identification élastique positionnée de façon amovible et maintenue par friction sur le support de cartouche ou l'extrémité proximale du dispositif et présentant une surface extérieure qui est un identifiant du médicament contenu dans la cartouche.

2. Système d'identification selon la revendication 1, dans lequel la surface extérieure de la bande est pigmentée de façon à permettre à un utilisateur du dispositif d'injection d'identifier et de distinguer visuellement le dispositif comme contenant un type spécifique de médicament.

3. Système d'identification selon la revendication 1, dans lequel la surface extérieure de la bande est texturée de façon à permettre à un utilisateur du dispositif d'injection d'identifier et de distinguer de façon tactile le dispositif comme contenant un type spécifique de médicament.

4. Système d'identification de médicaments pour un dispositif d'injection de doses multiples, comprenant:
a. un dispositif d'injection de type crayon avec des extrémités distale et proximale;
b. un support de cartouche placé à l'extrémité distale, configuré pour accepter une cartouche de médicament; et
c. une attache de poche fixée à l'extrémité proximale du dispositif ou à un capuchon amovible couvrant le support de cartouche, présentant une conception de structure qui est un identifiant du médicament contenu dans la cartouche.

5. Système d'identification selon la revendication 4, dans lequel la conception de structure de l'attache de poche est configurée de façon à identifier et distinguer de façon tactile le dispositif comme contenant un type spécifique de médicament.

6. Système d'identification selon la revendication 4, dans lequel l'attache est fixée de façon amovible au dispositif d'injection de façon à permettre à un utilisateur d'échanger différentes attaches de poche ayant des conceptions de structure différentes.
